Europäisches Patentamt
European Patent Office
Office européen des brevets

(19)

(11) Publication number: 0 174 833
A2

(12) # EUROPEAN PATENT APPLICATION

(21) Application number: 85306429.3

(22) Date of filing: 10.09.85

(51) Int. Cl.$^4$: C 07 D 471/04
A 61 K 31/47
//(C07D471/04, 249:00, 221:00)

(30) Priority: 11.09.84 GB 8422918

(43) Date of publication of application:
19.03.86 Bulletin 86/12

(84) Designated Contracting States:
AT BE CH DE FR GB IT LI LU NL SE

(71) Applicant: MAY & BAKER LIMITED

Dagenham Essex, RM10 7 XS(GB)

(72) Inventor: McClenaghan, Ian
May & Baker Limited
Dagenham Essex RM10 7XS(GB)

(72) Inventor: Saunders, Libert Clinton
May & Baker Limited
Dagenham Essex RM10 7XS(GB)

(74) Representative: Bentham, Stephen et al,
J.A. Kemp & Co. 14 South Square Gray's Inn
London WC1R 5EU(GB)

(54) **Triazoloquinoline derivatives.**

(57) Triazoloquinoline derivatives of the general formula:

$R^1(NHCH=CR^2R^3)_n$ I

wherein $R^1$ represents a 1,2,4-triazolo[4,3-a]quinoline group which is unsubstituted or substituted by one or more substituents $R^4$ [which may be the same or different and each represents a halogen atom or an amino, carboxy, cyano, nitro, hydroxy, formyl, trifluoromethyl, aryl, aryloxy, arylthio, benzyloxycarbonylamino, sulphamoyl, tetrazol-5-yl, carbamoyl, thiocarbamoyl, arylcarbamoyl or aroyl group, or a straight- or branched-chain alkyl group containing from1 to 6 carbon atoms, or a straight- or branched-chain alkoxy, alkylthio, alkylsulphinyl, alkylsulphonyl, alkylamino, alkylsulphamoyl or arylalkyl group containing from 1 to 6 carbon atoms in the alkyl moiety, a straight- or branched-chain alkanoyl, alkoxycarbonyl, alkoxycarbonylamino, alkylcarbamoyl or alkanoylamino group containing from 2 to 6 carbon atoms, an *N*-benzyloxycarbonyl-*N*-alkylamino group wherein the alkylamino moiety is straight- or branched-chain and contains from 1 to 6 carbon atoms, or a dialkylsulphamoyl, dialkylamino or dialkylcarbamoyl group wherein the alkyl moieties may be straight or branched and may each contain from 1 to 6 carbon atoms and may be linked together to form a ring], $R^2$ and $R^3$ may be the same or different and each represents a cyano group, a straight- or branched-chain alkoxycarbonyl group containing from 2 to 6 carbon atoms, or a phenylsulphonyl group which may be unsubstituted or substituted by one or more substituents $R^4$ (as hereinbefore defined), aryl groups and moieties and aroyl groups within the definition of $R^4$ optionally bearing one or more substituents, and *n* represents 1 or 2 or, when an aryl group or moiety or an aroyl group represented by $R^4$ carries one or more substituents $-NHCH=CR^2R^3$ (wherein $R^2$ and $R^3$ are as hereinbefore defined), *n* represents 0, 1 or 2, and salts thereof, possess useful pharmacological properties.

EP 0 174 833 A2

0174833

DESCRIPTION

"TRIAZOLOQUINOLINE DERIVATIVES"

This invention relates to new therapeutically useful triazoloquinoline derivatives, to a process for their preparation, to pharmaceutical compositions containing them, and to their use as pharmaceuticals.

The new triazoloquinoline derivatives are compounds of the general formula:-

$$R^1(NHCH=CR^2R^3)_{\underline{n}} \qquad I$$

wherein $R^1$ represents a 1,2,4-triazolo[4,3-a]-quinoline group which is unsubstituted or substituted by one or more substituents $R^4$ [which may be the same or different and each represents a halogen (i.e. fluorine, chlorine, bromine or iodine) atom or an amino, carboxy, cyano, nitro, hydroxy, formyl, trifluoromethyl, aryl, aryloxy, arylthio, benzyloxycarbonylamino, sulphamoyl, tetrazol-5-yl, carbamoyl, thiocarbamoyl, arylcarbamoyl or aroyl group, or a straight- or branched-chain alkyl group containing from 1 to 6 carbon atoms, or a straight- or branched-chain alkoxy, alkylthio, alkylsulphinyl, alkylsulphonyl, alkylamino, alkylsulphamoyl or arylalkyl group containing from 1 to 6 carbon atoms in the alkyl moiety, a straight- or branched-chain alkanoyl, alkoxycarbonyl, alkoxycarbonylamino,

alkylcarbamoyl or alkanoylamino group containing from 2 to 6 carbon atoms, an N-benzyloxycarbonyl-N-alkylamino group wherein the alkylamino moiety is straight- or branched-chain and contains from 1 to 6 carbon atoms, or a dialkylsulphamoyl, dialkylamino or dialkylcarbamoyl group wherein the alkyl moieties may be straight or branched and may each contain from 1 to 6 carbon atoms and may be linked together to form a ring, preferably a 5- to 9-membered ring], $R^2$ and $R^3$ may be the same or different and each represents a cyano group, a straight- or branched-chain alkoxycarbonyl group containing from 2 to 6 carbon atoms, or a phenylsulphonyl group which may be unsubstituted or substituted by one or more substituents $R^4$ (as hereinbefore defined) aryl groups and moieties and aroyl groups within the definition of $R^4$ being, for example, phenyl or benzoyl groups and optionally bearing one or more substituents, for example substituents selected from those listed above in the definition of $R^4$ and from groups of the formula $-NHCH=CR^2R^3$ wherein $R^2$ and $R^3$ are as hereinbefore defined, _n_ represents 1 or 2 or, when one or more substituents $R^4$ carries one or more groups $-NHCH=CR^2R^3$ (as hereinbefore defined), _n_ represents 0, 1 or 2 and pharmaceutically acceptable salts thereof.

The substituents $R^4$ may be the same or different.

Preferred compounds of formula I include those wherein the values of $R^1$ include 1,2,4-triazolo[4,3-a]quinol-7-yl groups unsubstituted or substituted by one or two substituents $R^4$ as hereinbefore defined, more particularly two substituents in the 1- and 5-positions, for example compounds of the general formula shown in Figure II of the drawings assembled at the end of the present specification, wherein $R^2$ and $R^3$ are as hereinbefore defined, $R^5$ represents a straight- or branched-chain alkyl group containing from 1 to 6 carbon atoms and $R^6$ represents a phenyl group which is unsubstituted or substituted by one or more straight- or branched-chain alkyl, alkoxy or alkylthio groups containing from 1 to 6 carbon atoms.

When $R^2$ and $R^3$ are different, this specification is intended to embrace the E-form and the Z-form, which arise as a result of geometrical isomerism, and mixtures thereof.

Especially important compounds of formula I are as follows:-

7-[2,2-bis(ethoxycarbonyl)ethen-1-ylamino]-5-methyl-1-phenyl-1,2,4-triazolo[4,3-a]quinoline; A

7-[2,2-bis(ethoxycarbonyl)ethen-1-ylamino]-5-methyl-1-(4-methylphenyl)-1,2,4-triazolo-[4,3-a]quinoline; B

7-[2,2-bis(ethoxycarbonyl)ethen-1-ylamino]-1-(4-methoxyphenyl)-5-methyl-1,2,4-triazolo-[4,3-a]quinoline; C

1-(4-tert-butylphenyl)-7-[2,2-bis(ethoxycarbonyl)ethen-1-ylamino]-5-methyl-1,2,4-triazolo[4,3-a]quinoline D

1-[4-{2,2-bis(ethoxycarbonyl)ethen-1-ylamino}phenyl]-5-methyl-1,2,4-triazolo[4,3-a]-quinoline; E

7-[2,2-bis(ethoxycarbonyl)ethen-1-ylamino]-1-[3-{2,2-bis(ethoxycarbonyl)ethen-1-ylamino}phenyl]-5-methyl-1,2,4-triazolo[4,3-a]-quinoline; F

1-(4-methoxyphenyl)-5-methyl-7-[2,2-bis(phenylsulphonyl)ethen-1-ylamino]-1,2,4-triazolo[4,3-a]-quinoline; G

1-(4-methoxyphenyl)-5-methyl-7-(2,2-dicyanoethen-1-ylamino)-1,2,4-triazolo[4,3-a)quinoline; and H

7-[2,2-bis(ethoxycarbonyl)ethen-1-ylamino-1-(4-methylthiophenyl)-5-methyl-1,2,4-triazolo[4,3-a]-quinoline. I

Compounds A, B, C and D are of particular importance.

The letters A to I are allocated to the compounds for easy reference later in the specification, e.g. in the Tables and in the Examples.

The compounds have valuable pharmacological properties, in particular properties which are indicative of utility in the treatment of arthritic disorders such as rheumatoid arthritis.

For example, from tests in mice it was calculated that compounds of formula I, when twice administered orally, one hour before and four hours after the administration of antigen, each time at the doses shown in the following Table I, would reduce by 50% the inhibition of migration of incubated mouse macrophage cells measured in a manner similar to that described by Likhite et al., Cellular Immunology, Vol. 5, 1972, page 377. This is a measure of antagonism or reduction of the levels of lymphokines and is indicative of utility in the treatment of arthritic patients.

TABLE 1

| Test Compound | oral ED50 dose (mg/kg animal body weight) |
| --- | --- |
| A | 7 |
| C | less than 10 |

Furthermore, in laboratory tests, the compounds have been shown to inhibit the deterioration of joints in the limbs of cavies and rats. These results are particularly important because compounds currently employed in the treatment of arthritic disorders are primarily antiinflammatories and do not possess the said ability to inhibit joint deterioration.

The compounds also exhibit properties which are indicative of utility in the treatment of ailments such as allergy, asthma, inflammatory diseases, cerebral ischaemia, myocardial ischaemia and psoriasis.

For example, in tests *in vitro* at concentrations of 0.0001M, compounds of formula I inhibited lipoxygenase by the percentages shown in the following Table II.

TABLE II

| Test Compound | % inhibition of lipoxygenase |
|---|---|
| A | 100 |
| B | 100 |
| | 83 |
| D | 98 |
| E | 50 |

The compounds of formula I also possess valuable immunomodulatory activity and are of use in the treatment of organ grafts and skin grafts and in the treatment of immunological diseases.

The beneficial properties of the compounds of formula I are enhanced by the fact that they have only very low mammalian toxicity.

The compounds of formula I may be prepared by the application or adaptation of known methods.

Thus as a feature of the present invention, the compounds of formula I are prepared by the reaction of compounds of the general formula:-

$$R^1(NH_2)_n \qquad III$$

(wherein $R^1$ and $n$ are as hereinbefore defined) with compounds of the general formula:-

$$R^7OCH{=}CR^2R^3 \qquad IV$$

(wherein $R^2$ and $R^3$ are as hereinbefore defined and $R^7$ represents an alkyl group of 1 to 5 carbon atoms,

preferably with a straight chain, e.g. ethyl), generally at temperatures between 0° and 170°C, preferably at or near 100°C, optionally in the presence of an inert solvent. Suitable inert solvents include aromatic hydrocarbons, e.g. toluene, aliphatic ethers, aliphatic nitriles, aliphatic amides, e.g. N,N-dimethylacetamide, and aliphatic alcohols, e.g. ethanol.

As will be evident to those skilled in the art, the process for the preparation of compounds of formula I by the reaction together of compounds of formulae III and IV is liable to lead to mixtures of products if the value of one or more of the substituents $R^4$ is such that side reactions can occur, for example if an amino, hydroxy or alkylamino group is present. In such circumstances it may be preferably to prepare the desired compound of formula I by the interconversion of another compound of formula I. For example, amino groups can be formed by reduction of nitro groups, hydroxy groups can be formed by the reduction of benzyloxy groups, and alkylamino groups can be formed by the reduction of N-benzyloxycarbonyl-N-alkylamino groups.

Thus, as a feature of the present invention, compounds of formula I wherein at least one of the substituents $R^4$ represents or contains an amino,

hydroxy or straight- or branched-chain alkylamino group containing from 1 to 6 carbon atoms, any other substituents being as hereinbefore defined, are prepared by the conversion of the corresponding compound of formula I wherein at least one of the substituents $R^4$ represents or contains a nitro, benzyloxy or N-benzyloxycarbonyl-N-alkylamino group wherein the alkylamino moiety is straight- or branched-chain and contains from 1 to 6 carbon atoms, respectively, any other substituents being as hereinbefore defined. The reaction may be carried out by the application or adaptation of known methods, for example by hydrogenation in the presence of a hydrogenation catalyst, e.g. palladium on charcoal (e.g. 5-10% w/w), usually in a solvent such as a lower alkanol, e.g. ethanol, dimethylformamide, dioxan or acetic acid.

Compounds of formula III may be prepared by the application or adaptation of known methods, for example by the reduction of compounds of the general formula:-

$$R^1(NO_2)_n \qquad V$$

wherein $R^1$ and $n$ are as hereinbefore defined. The reaction may be carried out by the application or adaptation of known methods, for example by hydrogenation in the presence of a hydrogenation

catalyst, e.g. palladium on charcoal (e.g. 5-10% w/w), usually in solvent such as a lower alkanol, e.g. ethanol, dimethylformamide, dioxan or acetic acid.

Compounds of formula V may be prepared by the application or adaptation of known methods. For example, compounds of the general formula shown in Figure VI of the drawings (wherein $R^5$ and $R^6$ are as hereinbefore defined) may be prepared by one of the following two methods A and B:-

A. The reaction together of compounds of the general formula shown in Figure VII of the drawings (wherein $R^5$ is as hereinbefore defined and X represents a halogen, e.g. chlorine, atom) and compounds of the general formula:-

$$R^6CONHNH_2 \qquad VIII$$

(wherein $R^6$ is as hereinbefore defined) at an elevated temperature, e.g. between 200° and 300°C, preferably in a suitable solvent, e.g. Diphyl [i.e. the eutectic mixture of diphenyl ether and diphenyl (approximately 3:1), b.p. 258°C].

B. The oxidation of compounds of the general formula shown in Figure IX of the drawings (wherein $R^5$ and $R^6$ are as hereinbefore defined), for example by means of a ferric salt, e.g. ferric chloride, preferably at a temperature between 0° and 100°C, preferably in a suitable solvent, e.g. a lower alkanol, e.g. ethanol.

By the term "pharmaceutically acceptable salt" in relation to compounds of general formula I is meant a salt formed by reaction with an acid by the application or adaptation of known methods or by reaction with a base by the application or adaptation of known methods so that the anion (in the case of an acid addition salt) or the cation (in the case of a salt formed with a pharmaceutically acceptable base) is relatively innocuous to the animal organism when used in therapeutic doses so that the beneficial pharmacological properties of the parent compound of general formula I are not vitiated by side-effects ascribable to the said anion or cation.

Suitable acid addition salts include salts derived from inorganic acids, for example hydrochlorides, hydrobromides, phosphates, sulphates and nitrates, and organic salts, for example methanesulphonates, 2-hydroxyethanesulphonates, oxalates, lactates, tartrates, acetates, salicylates, citrates, propionates, succinates, fumarates, maleates, methylene-bis-ß-hydroxynaphthoates, gentisates and di-*p*-toluoyltartrates.

Suitable salts formed by compounds of general formula I with pharmaceutically acceptable bases include the alkali metal (e.g. sodium and potassium) alkaline earth metal (e.g. calcium and magnesium), and ammonium salts, and salts of amines known in the art to be pharmaceutically acceptable, e.g. ethylene

diamine, choline, diethanolamine, triethanolamine, octadecylamine, diethylamine, triethylamine, 2-amino-2-(hydroxymethyl)propane-1,3-diol and 1-(3,4-dihydroxyphenyl)-2-isopropylaminoethanol.

It is to be understood that, where in this specification reference is made to compounds of general formula I, it is intended to refer also to their pharmaceutically acceptable salts as indicated above, where the context so permits.

Compounds of the formulae IV, VII, VIII and IX may be prepared by the application or adaptation of known methods.

By the term "known methods" as used in this specification is meant methods heretofore used or described in the literature.

The following Examples illustrate the preparation of the compounds of the present invention and the Reference Examples illustrate the preparation of intermediates.

EXAMPLE 1

Compounds A, B, C, D, E and F

A mixture of 7-amino-5-methyl-1-phenyl-1,2,4-triazolo[4,3-a]quinoline (7.1g; prepared as described in Reference Example 1) and 2-ethoxy-1,1-bis-(ethoxycarbonyl)ethene (25ml) was heated on a steam bath for 3 hours. It was then cooled to room temperature, triturated with diethyl ether and the solid was recrystallised from ethyl acetate, to give 7-[2,2-bis(ethoxycarbonyl)ethen-1-ylamino]-5-methyl-1-phenyl-1,2,4-triazolo[4,3-a]quinoline (6.3g), m.p. 199-201°C.

By proceeding in a similar manner, but replacing the 7-amino-5-methyl-1-phenyl-1,2,4-triazolo-[4,3-a]quinoline, used as a starting material, by the appropriate quantities of:-

7-amino-5-methyl-1-(4-methylphenyl)-1,2,4-triazolo[4,3-a]quinoline;

7-amino-1-(4-methoxyphenyl)-5-methyl-1,2,4-triazolo[4,3-a]quinoline;

7-amino-1-(4-tert-butylphenyl)-5-methyl-1,2,4-triazolo[4,3-a]quinoline;

1-(4-aminophenyl)-5-methyl-1,2,4-triazolo-[4,3-a]quinoline; and

7-amino-1-(3-aminophenyl)-5-methyl-1,2,4-triazolo[4,3-a]quinoline;

respectively, all prepared as described in Reference Example 1, there were prepared:-

7-[2,2-bis(ethoxycarbonyl)ethen-1-ylamino]-5-methyl-1-(4-methylphenyl)-1,2,4-triazolo[4,3-a]-quinoline, m.p. 222-225°C [purified by recrystallisation from ethyl acetate followed by medium pressure chromatography on silica gel, eluting with a mixture of chloroform and methanol (9:1 v/v)];

7-[2,2-bis(ethoxycarbonyl)ethen-1-ylamino]-1-(4-methoxyphenyl)-5-methyl-1,2,4-triazolo[4,3-a]-quinoline, m.p. 214-218°C [purified by recrystallisation from ethyl acetate followed by medium pressure chromatography on silica gel, eluting with a mixture of chloroform and methanol (9:1 v/v)];

1-(4-tert-butylphenyl)-7-[2,2-bis(ethoxycarbonyl)ethen-1-ylamino]-5-methyl-1,2,4-triazolo-[4,3-a]quinoline, m.p. 219-222°C [subjected to medium pressure chromatography on silica gel, eluting with a mixture of chloroform and methanol (9:1 v/v), followed by a similar chromatography eluting with chloroform];

1-[4-{2,2-bis(ethoxycarbonyl)ethen-1-ylamino}-phenyl]-5-methyl-1,2,4-triazolo[4,3-a]quinoline, m.p. 193°C (recrystallised from ethanol); and

7-[2,2-bis(ethoxycarbonyl)ethen-1-ylamino]-1-[3-{2,2-bis(ethoxycarbonyl)ethen-1-ylamino}phenyl]-5-methyl-1,2,4-triazolo[4,3-a]quinoline, m.p. 206-208°C (recrystallised from ethyl acetate).

EXAMPLE 2

Compound G

A mixture of 7-amino-1-(4-methoxyphenyl)-5-methyl-1,2,4-triazolo[4,3-a]quinoline (1.01g) and 1,1-bis(phenylsulphonyl)-2-ethoxyethene (1.8g) in dry N,N-dimethylacetamide (20 ml) was heated at reflux for 2.25 hours. The mixture was poured onto ice (75g) and the resulting pinkish orange solid was filtered off, washed with water, with boiling ethanol (100ml), and with aqueous ethanol, to give 1-(4-methoxyphenyl)-5-methyl-7-[2,2-bis(phenylsulphonyl)ethen-1-ylamino]-1,2,4-triazolo[4,3-a]quinoline (1.15g), m.p. 220-222°C.

EXAMPLE 3

Compound H

By proceeding in a manner similar to that described in Example 2 but replacing the 1,1-bis(phenylsulphonyl)-2-ethoxyethene, used as a starting material, by the appropriate quantity of 1,1-dicyano-2-ethoxyethene, there was prepared 1-(4-methoxyphenyl)-5-methyl-7-(2,2-dicyanoethen-1-ylamino)-1,2,4-triazolo[4,3-a)-quinoline, m.p. 307°C (from aqueous dimethylformamide).

EXAMPLE 4

Compound I

By proceeding in a manner similar to that described in the foregoing Examples there was prepared 7-[2,2-bis(ethoxycarbonyl)ethen-1-ylamino-1-(4-methylthiophenyl)-5-methyl-1,2,4-triazolo[4,3-a]-quinoline, m.p. 218-220°C.

REFERENCE EXAMPLE 1

A solution of 5-methyl-7-nitro-1-phenyl-1,2,4-triazolo[4,3-a]quinoline (32.5g; prepared as described in Reference Example 2) in glacial acetic acid (415ml) was hydrogenated over a catalyst of palladium on charcoal (5% w/w; 4g). When absorption of hydrogen was complete the catalyst was filtered off and the solvent was removed on a rotary evaporator. The resulting crude solid was crystallised from ethoxyethanol, to give 7-amino-5-methyl-1-phenyl-1,2,4-triazolo[4,3-a]quinoline (27.2g), m.p. 298-300°C.

By proceeding in a similar manner, but replacing the 5-methyl-7-nitro-1-phenyl-1,2,4-triazolo-[4,3-a]quinoline, used as starting material, by the appropriate quantities of:-

5-methyl-1-(4-methylphenyl)-7-nitro-1,2,4-triazolo[4,3-a]quinoline, prepared as described in Reference Example 3;

1-(4-methoxyphenyl)-5-methyl-7-nitro-1,2,4-triazolo[4,3-a]quinoline, prepared as described in Reference Example 3;

1-(4-tert-butylphenyl)-5-methyl-7-nitro-1,2,4-triazolo[4,3-a]quinoline, prepared as described in Reference Example 4;

5-methyl-1-(4-nitrophenyl)-1,2,4-triazolo-[4,3-a]quinoline, prepared as described in Reference Example 2; and

5-methyl-7-nitro-1-(3-nitrophenyl)-1,2,4-triazolo[4,3-a]quinoline, prepared as described in Reference Example 3;

respectively there were prepared:-

7-amino-5-methyl-1-(4-methylphenyl)-1,2,4-triazolo[4,3-a]quinoline, m.p. 231-233°C [purified as follows:- The crude solid was treated with a mixture of concentrated hydrochloric acid and water (1:2 v/v;180ml) for 45 minutes, and the mixture was then treated with charcoal, filtered, cooled, and treated with excess aqueous sodium hydroxide solution (50% w/v). The resulting precipitate was washed with water and recrystallised from methanol];

7-amino-1-(4-methoxyphenyl)-5-methyl-1,2,4-triazolo[4,3-a]quinoline, m.p. 230-232°C [purified in a manner similar to that described immediately heretofore but recrystallising twice, from ethanol];

7-amino-1-(4-tert-butylphenyl)-5-methyl-1,2,4-triazolo[4,3-a]quinoline, m.p. 245-248°C [purified in a manner similar to that described immediately heretofore but recrystallising once, from isopropanol];

1-(4-aminophenyl)-5-methyl-1,2,4-triazolo-[4,3-a]quinoline, m.p. 246-248°C; and

7-amino-1-(3-aminophenyl)-5-methyl-1,2,4-triazolo[4,3-a]quinoline, m.p. 312-316°C.

REFERENCE EXAMPLE 2

A stirred mixture of 2-chloro-4-methyl-6-nitroquinoline (23.4g), benzoylhydrazine (13.6g) and Diphyl (300ml) was heated to 250°C and maintained at that temperature for 15 minutes, and then it was then left to cool to room temperature. A quantity of solid (18.1g) separated and was filtered off. A further quantity of solid (6.6g) was obtained by dilution of the mother liquors with light petroleum. The combined solids were recrystallised from ethanol, with the aid of charcoal, to give 5-methyl-7-nitro-1-phenyl-1,2,4-triazolo[4,3-a]quinoline (10.8g), m.p. 256-258°C (with decomposition).

By proceeding in a similar manner, but replacing the starting material by the appropriate quantities of 2-chloro-4-methylquinoline and 4-nitrobenzoylhydrazine,there was prepared:-

5-methyl-1-(4-nitrophenyl)-1,2,4-triazolo-[4,3-a]quinoline, m.p. 282-284°C (recrystallised from dimethylformamide).

REFERENCE EXAMPLE 3

A stirred mixture of 2-(4-methoxybenzylidenehydrazino)-4-methyl-6-nitroquinoline (63.6g) and ethanol (500ml) at the reflux temperature was treated gradually with a solution of ferric chloride hydrate

(23 g) in ethanol (250ml) during a period of 10 minutes, and the mixture was heated at reflux for a further period of 3 hours. The resulting solution was poured into water and a solid was precipitated. The solid was filtered off and washed with water, to give 1-(4-methoxyphenyl)-5-methyl-7-nitro-1,2,4-triazolo[4,3-a]-quinoline (48.7g), m.p. 277-279°C.

By proceeding in a similar manner, but using the appropriate quantity of 2-(4-methylbenzylidene-hydrazino)-4-methyl-6-nitroquinoline as starting material, there was prepared:-

5-methyl-1-(4-methylphenyl)-7-nitro-1,2,4-triazolo[4,3-a]quinoline, m.p. 278-281°C.

By again proceeding in a similar manner, but using the appropriate quantity of 2-(3-nitro-benzylidenehydrazino)-4-methyl-6-nitroquinoline as starting material, there was prepared:-

5-methyl-7-nitro-1-(3-nitrophenyl)-1,2,4-triazolo[4,3-a]quinoline, m.p. 254-256°C.

## REFERENCE EXAMPLE 4

A stirred solution of 2-chloro-4-methyl-6-nitroquinoline (60g) and 4-tert-butylbenzoyl-hydrazine (51.8g) in Diphyl (500ml) was heated at 210°C for 90 minutes. It was then cooled to room temperature, treated with concentrated

sulphuric acid (100ml) and shaken. The sulphuric acid layer was separated and poured into a mixture of ice and water, and the resulting solid was filtered off, washed with water and recrystallised from ethanol, to give 1-(4-tert-butylphenyl)-5-methyl-7-nitro-1,2,4-triazolo[4,3-a]quinoline (40.4g), m.p. 296-299°C.

The present invention includes within its scope pharmaceutical compositions which comprise at least one compound of formula I in association with a pharmaceutically acceptable carrier or coating. In clinical practice the compositions of the present invention will normally be administered orally or rectally, or parenterally, for example topically or intraarticularly.

Solid compositions for oral administration include compressed tablets, pills, dispersible powders, and granules. In such solid compositions one or more of the active compounds is mixed with at least one inert diluent such as calcium carbonate, potato starch, alginic acid, or lactose. The compositions may also comprise, as is normal practice, additional substances other than inert diluents, e.g. lubricating agents, such as magnesium stearate. Liquid compositions for oral administration include pharmaceutically acceptable emulsions, solutions, suspensions, and elixirs containing inert diluents commonly used in the art, such as

water and liquid paraffin. Besides inert diluents such compositions may also comprise adjuvants, such as wetting and suspending agents, and sweetening, flavouring, perfuming and preserving agents.

The compositions according to the invention, for oral administration, also include capsules of absorbable material such as gelatin containing one or more of the active compounds with or without the addition of diluents or excipients.

Solid compositions for rectal administration include suppositories formulated in manner known *per se* and containing one or more of the active compounds.

Preparations according to the invention for parenteral administration include sterile aqueous or non-aqueous solutions, suspensions, or emulsions. Examples of non-aqueous solvents or suspending media are propylene glycol, vegetable oils such as olive oil, and injectable organic esters such as ethyl oleate. These compositions may also contain adjuvants such as preserving, wetting, emulsifying and dispersing agents. They may be sterilised, for example, by filtration through a bacteria-retaining filter, by incorporation of sterilising agents in the compositions or by irradiation. They may also be manufactured in the form of sterile solid

compositions, which can be dissolved in a sterile injectable medium immediately before use. As well as the more customary intravenous and intramuscular routes, the compositions may be administered by intraarticular injection.

Compositions in the form of solutions or suspensions, if desired together with additives as described above, in water or in vegetable or other greases, paraffin or other waxes, or lacquers or creams or lotions, to be applied topically, for example to the skin area around an affected joint to relieve arthritis, are also included in the invention. They may also include additives such as nicotinamide to assist absorption.

The percentages of active ingredient in the compositions of the invention may be varied, it being necessary that they should constitute a proportion such that a suitable dosage for the desired effect shall be obtained. Obviously several unit dosage forms may be administered at about the same time. Generally the compositions should contain 0.1% to 80% by weight of active ingredient, especially when in tablet form.

The dose employed depends upon the desired effect, the route of administration and the duration of the treatment. In the adult, the doses are

generally between 0.01 and 100mg (preferably orally between 0.1 and 10mg, or intramuscularly, intravenously or intraarticularly between 0.01 and 10mg) of the compounds of formula I per kg body weight per day.

The compounds of formula I may be administered each day or, according to the wishes of the medical practitioner, less often, e.g. weekly.

The present invention provides a method of treating disorders, e.g. arthritic disorders, in man which comprises administering to the patient an amount of a compound of formula I sufficient to combat the disorder.

The following Composition Example illustrates pharmaceutical compositions according to the present invention.

COMPOSITION EXAMPLE 1

Capsules for oral administration were made up in the usual manner by filling No. 2 size gelatin capsules each with 155 mg of the following composition:-

| | |
|---|---|
| 7-[2,2-bis(ethoxycarbonyl)ethen-1-yl]-5-methyl-1-phenyl-1,2,4-triazolo[4,3-a]quinoline | 50 mg |
| potato starch | 100 mg |
| magnesium stearate | 2.5 mg |
| Aerosil | 2.5 mg |

Similar compositions can be prepared by the use of any of the other compounds of formula I, for example compounds B to I hereinbefore identified.

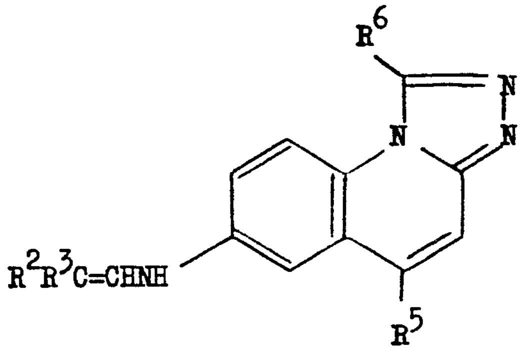

II

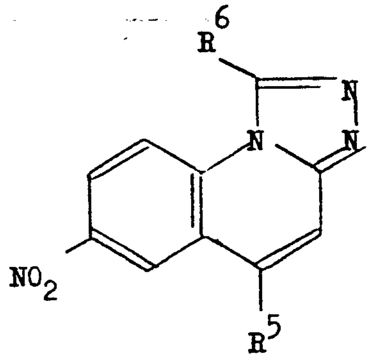

VI

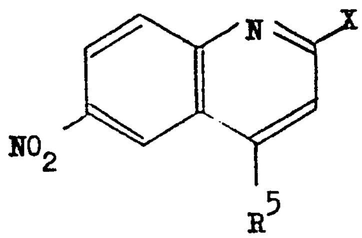

VII

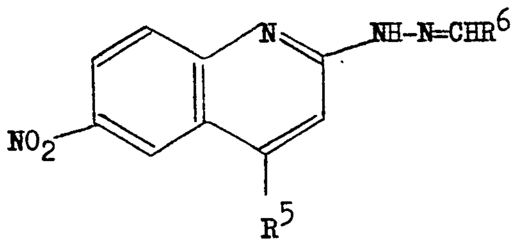

IX

CLAIMS

1. A triazoloquinoline derivative of the general formula:

$$R^1(NHCH{=}CR^2R^3)_n \quad \text{I}$$

wherein $R^1$ represents a 1,2,4-triazolo[4,3-a]quinoline group which is unsubstituted or substituted by one or more substituents $R^4$ [which may be the same or different and each represents a halogen atom or an amino, carboxy, cyano, nitro, hydroxy, formyl, trifluoromethyl, aryl, aryloxy, arylthio, benzyloxycarbonylamino, sulphamoyl, tetrazol-5-yl, carbamoyl, thiocarbamoyl, arylcarbamoyl or aroyl group, or a straight- or branched-chain alkyl group containing from 1 to 6 carbon atoms, or a straight- or branched-chain alkoxy, alkylthio, alkylsulphinyl, alkylsulphonyl, alkylamino, alkylsulphamoyl or arylalkyl group containing from 1 to 6 carbon atoms in the alkyl moiety, a straight- or branched-chain alkanoyl, alkoxycarbonyl, alkoxycarbonylamino, alkylcarbamoyl or alkanoylamino group containing from 2 to 6 carbon atoms, an _N_-benzyloxycarbonyl-_N_-alkylamino group wherein the alkylamino moiety is straight- or branched-chain and contains from 1 to 6 carbon atoms, or a dialkylsulphamoyl, dialkylamino or dialkylcarbamoyl group

wherein the alkyl moieties may be straight or branched and may each contain from 1 to 6 carbon atoms and may be linked together to form a ring], $R^2$ and $R^3$ may be the same or different and each represents a cyano group, a straight- or branched-chain alkoxycarbonyl group containing from 2 to 6 carbon atoms, or a phenylsulphonyl group which may be unsubstituted or substituted by one or more substituents $R^4$ (as hereinbefore defined), aryl groups and moieties and aroyl groups within the definition of $R^4$ optionally bearing one or more substituents, and _n_ represents 1 or 2 or, when an aryl group or moiety or an aroyl group represented by $R^4$ carries one or more substituents $-NHCH{=}CR^2R^3$ (wherein $R^2$ and $R^3$ are as hereinbefore defined), _n_ represents 0, 1 or 2, and pharmaceutically acceptable salts thereof.

2. A compound according to claim 1 wherein $R^1$ represents a 1,2,4-triazolo[4,3-a]quinol-7-yl group unsubstituted or substituted by one or two substituents $R^4$ as defined in claim 1.

3. A compound according to claim 1 wherein $R^1$ represents a 1,2,4-triazolo[4,3-a]quinol-7-yl group substituted by two substituents $R^4$, as defined in claim 1, in the 1- and 5-positions.

4. A compound according to claim 1 of the general formula:

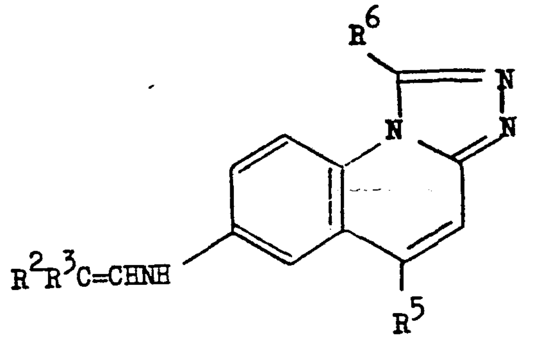

II

wherein $R^2$ and $R^3$ are as defined in claim 1, $R^5$ represents a straight- or branched-chain alkyl group containing from 1 to 6 carbon atoms and $R^6$ represents a phenyl group which is unsubstituted or substituted by one or more straight- or branched-chain alkyl, alkoxy or alkylthio groups containing from 1 to 6 carbon atoms.

5. A compound according to claim 1 which is
7-[2,2-bis(ethoxycarbonyl)ethen-1-ylamino]-5-methyl-1-phenyl-1,2,4-triazolo[4,3-a]quinoline,
7-[2,2-bis(ethoxycarbonyl)ethen-1-ylamino]-5-methyl-1-(4-methylphenyl)-1,2,4-triazolo[4,3-a]quinoline,
7-[2,2-bis(ethoxycarbonyl)ethen-1-ylamino]-1-(4-methoxyphenyl)-5-methyl-1,2,4-triazolo[4,3-a]quinoline,
1-(4-tert-butylphenyl)-7-[2,2-bis(ethoxycarbonyl)ethen-1-ylamino]-5-methyl-1,2,4-triazolo[4,3-a]quinoline,
1-[4-{2,2-bis(ethoxycarbonyl)ethen-1-ylamino}phenyl]-5-methyl-1,2,4-triazolo[4,3-a]quinoline,
7-[2,2-bis(ethoxycarbonyl)ethen-1-ylamino]-1-[3-{2,2-bis(ethoxycarbonyl)ethen-1-ylamino}phenyl]-5-methyl-1,2,4-triazolo[4,3-a]quinoline, 1-(4-methoxyphenyl)-5-methyl-7-[2,2-bis(phenylsulphonyl)-ethen-1-ylamino]-1,2,4-triazolo[4,3-a]quinoline, 1-(4-methoxyphenyl)-5-methyl-7-(2,2-dicyanoethen-1-ylamino)-1,2,4-triazolo[4,3-a]quinoline, or 7-[2,2-bis(ethoxycarbonyl)ethen-1-ylamino-1-(4-methylthiophenyl)-5-methyl-1,2,4-triazolo[4,3-a]quinoline.

6. A process for the preparation of a compound according to claim 1 which comprises the reaction of a compound of the general formula:

$$R^1(NH_2)_{\underline{n}} \qquad III$$

(wherein $R^1$ and $\underline{n}$ are as defined in claim 1) with a compound of the general formula:

$$R^7OCH=CR^2R^3 \qquad IV$$

wherein $R^2$ and $R^3$ are as defined in claim 1 and $R^7$ represents an alkyl group of 1 to 5 carbon atoms.

7. A process for the preparation of a compound according to claim 1 wherein at least one of the substituents $R^4$ represents or contains an amino, hydroxy or straight- or branched-chain alkylamino group containing from 1 to 6 carbon atoms, any other substituents being as defined in claim 1, by the conversion of a corresponding compound of formula I wherein at least one of the substituents $R^4$ represents or contains a nitro, benzyloxy or N-benzyloxycarbonyl-N-alkylamino group wherein the alkylamino moiety is straight- or branched-chain and contains from 1 to 6 carbon

atoms, respectively, any other substituents being as defined in claim 1.

8. A process according to claim 6 or 7 followed by the step of converting by methods known per se a triazoloquinoline derivative thus obtained into a pharmaceutically acceptable salt thereof.

9. A pharmaceutical composition which comprises, as active ingredient, a triazoloquinoline derivative according to claim 1, or a pharmaceutically acceptable salt thereof, in association with a pharmaceutically acceptable carrier or coating.

10. A triazoloquinoline derivative according to claim 1, or a pharmaceutically acceptable salt thereof, for use in the preparation of a pharmaceutical composition for the treatment of arthritic disorders, allergy, asthma, inflammatory diseases, cerebral ischaemia, myocardial ischaemia or psoriasis, or immunological diseases.

CLAIMS (AUSTRIAN)

1. A process for the preparation of a triazoloquinoline derivative of the general formula:

$$R^1(NHCH=CR^2R^3)_{\underline{n}} \quad \text{I}$$

wherein $R^1$ represents a 1,2,4-triazolo[4,3-a]quinoline group which is unsubstituted or substituted by one or more substituents $R^4$ [which may be the same or different and each represents a halogen atom or an amino, carboxy, cyano, nitro, hydroxy, formyl, trifluoromethyl, aryl, aryloxy, arylthio, benzyloxycarbonylamino, sulphamoyl, tetrazol-5-yl, carbamoyl, thiocarbamoyl, arylcarbamoyl or aroyl group, or a straight- or branched-chain alkyl group containing from 1 to 6 carbon atoms, or a straight- or branched-chain alkoxy, alkylthio, alkylsulphinyl, alkylsulphonyl, alkylamino, alkylsulphamoyl or arylalkyl group containing from 1 to 6 carbon atoms in the alkyl moiety, a straight- or branched-chain alkanoyl, alkoxycarbonyl, alkoxycarbonylamino, alkylcarbamoyl or alkanoylamino group containing from 2 to 6 carbon atoms, an N-benzyloxycarbonyl-N-alkylamino group wherein the alkylamino moiety is straight- or branched-chain and contains from 1 to 6 carbon atoms, or a dialkylsulphamoyl, dialkylamino or dialkylcarbamoyl group wherein the alkyl moieties may be straight or branched and may each contain

from 1 to 6 carbon atoms and may be linked together to form a ring], $R^2$ and $R^3$ may be the same or different and each represents a cyano group, a straight- or branched-chain alkoxycarbonyl group containing from 2 to 6 carbon atoms, or a phenylsulphonyl group which may be unsubstituted or substituted by one or more substituents $R^4$ (as hereinbefore defined), aryl groups and moieties and aroyl groups within the definition of $R^4$ optionally bearing one or more substituents, and _n_ represents 1 or 2 or, when an aryl group or moiety or an aroyl group represented by $R^4$ carries one or more substituents $-NHCH=CR^2R^3$ (wherein $R^2$ and $R^3$ are as hereinbefore defined), _n_ represents 0, 1 or 2, or a pharmaceutically acceptable salt thereof, which process comprises

(A) the reaction of a compound of the general formula:

$$R^1(NH_2)_n \qquad \text{III}$$

(wherein $R^1$ and _n_ are as hereinbefore defined) with a compound of the general formula:

$$R^7OCH=CR^2R^3 \qquad \text{IV}$$

wherein $R^2$ and $R^3$ are as hereinbefore defined and $R^7$ represents an alkyl group of 1 to 5 carbon atoms, or

(B) when at least one of the substituents $R^4$ represents or contains an amino, hydroxy or straight- or branched-chain alkylamino group containing from 1 to 6 carbon atoms, any other substituents being as defined in claim 1, by the conversion of a corresponding compound of formula I wherein at least one of the substituents $R_4$

represents or contains a nitro, benzyloxy or N-benzyloxycarbonyl-N-alkylamino group wherein the alkylamino moiety is straight- or branched-chain and contains from 1 to 6 carbon atoms, respectively, any other substituents being as defined in claim 1, optionally followed by the step of converting by methods known per se a triazoloquinoline derivative thus obtained into a pharmaceutically acceptable salt thereof.

2. A process according to claim 1 wherein the reaction of the compound of general formula III with the compound of general formula IV is carried out at or near 100°C, optionally in the presence of an inert solvent.

3. A process according to claim 1 or 2 wherein $R^1$ represents a 1,2,4-triazolo[4,3-a]quinol-7-yl group unsubstituted or substituted by one or two substituents $R^4$ as defined in claim 1.

4. A process according to claim 1 or 2 wherein $R^1$ represents a 1,2,4-triazolo[4,3-a]quinol-7-yl group substituted by two substituents $R^4$ as defined in claim 1 in the 1- and 5-positions.

5. A process according to claim 1 or 2 wherein the compound of general formula I conforms to the general formula:

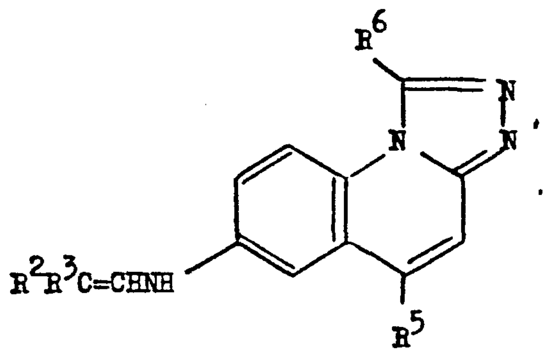

II

wherein $R^2$ and $R^3$ are as defined in claim 1, $R^5$ represents a straight- or branched-chain alkyl group containing from 1 to 6 carbon atoms and $R^6$ represents a phenyl group which is unsubstituted or substituted by one or more straight- or branched-chain alkyl, alkoxy or alkylthio groups containing from 1 to 6 carbon atoms.

6. A triazoloquinoline derivative of general fórmula I depicted in claim 1 or a pharmaceutically acceptable salt thereof, for use in the preparation of a pharmaceutical composition for the treatment of arthritic disorders, allergy, asthma, inflammatory diseases, cerebral ischaemia, myocardial ischaemia or psoriasis, or immunological diseases.